# EUROPÄISCHE PATENTSCHRIFT

(11) **EP 3 103 774 B1**
(45) Veröffentlichungstag und Bekanntmachung des Hinweises auf die Patenterteilung: **28.10.2020**
(21) Anmeldenummer: 16167194.6
(22) Anmeldetag: 27.04.2016
(51) Int. Cl.: C02F 3/34, C05F 17/00

(54) **VERFAHREN ZUM HERSTELLEN EINER BIOLOGISCH AKTIVEN, MIKROORGANISMEN ENTHALTENDEN WÄSSRIGEN LÖSUNG BZW. SUSPENSION**
A METHOD FOR THE PREPARATION OF A SOLUTION OR WATER SUSPENSION COMPRISING BIOLOGICALLY ACTIVE MICROORGANISMS
PROCEDE DE FABRICATION D'UNE SOLUTION OU SUSPENSION AQUEUSE CONTENANT DES MICROORGANISMES BIOLOGIQUEMENT ACTIFS

(30) Priorität: 10.06.2015 DE 102015109233
(43) Veröffentlichungstag der Anmeldung: 14.12.2016
(73) Patentinhaber: Lehmann, Stephan, 14193 Berlin (DE)
(72) Erfinder: Lehmann, Stephan, 14193 Berlin (DE)
(74) Vertreter: Raffay & Fleck

(56) Entgegenhaltungen:
- WO-A2-2012/122530
- INGHAM E R ED - INGHAM E R: "THE COMPOST TEA BREWING MANUAL", 1. April 2005 (2005-04-01), THE COMPOST TEA BREWING MANUAL, SOIL FOODWEB INCORPORATED, CORVALLIS, OREGON, US, PAGE(S) I - IX,1, XP008146491, * Seite ix, Absatz 3 - Absatz 3 * * Seite 2, letzter Absatz - Seite 3, Absatz 1 * * Seite 44, letzter Absatz - Seite 45, Absatz 1 * * Seite 4 und 5 "anaerobic conditions" * * Seite 37, Absatz 4 * * Seite 23 * * Seite 40 * * Seite 54; Tabelle 9 * * Seite 19, Absatz 1 *
- Cortesia Sanctuary ET AL: "How to Make Compost Tea", , 22. März 2013 (2013-03-22), XP055287653, Gefunden im Internet: URL:http://www.homecompostingmadeeasy.com/ composttea.html [gefunden am 2016-07-12]
- NORMAN Q ARANCON ET AL: "Vermicompost tea production and plant growth impacts", BIOCYCLE., Bd. 48, Nr. 11, 1. November 2007 (2007-11-01), Seite 51, XP055287454, US ISSN: 0276-5055
- Hassan Allahyari ET AL: "International Journal of Farming and Allied Sciences The process of production compost tea and its usage in agriculture: a review", , 1. Januar 2015 (2015-01-01), Seiten 2322-4134, XP055287890, Gefunden im Internet: URL:http://ijfas.com/wp-content/uploads/20 15/03/171-176.pdf [gefunden am 2017-07-12]
- Maria Gomez-Brandon: "effects of compost and vermicompost teas as organic fertilisers", , 1. Januar 2015 (2015-01-01), XP055295365, Gefunden im Internet: URL:www.researchgate.net/publication/27057 6602_Effects_of_compost_and_vermicompost_t eas_as_organic_fertilisers [gefunden am 2016-08-12]
- anonymous: "Anaerobic Composting", found in google search for "anaerobic compost tea airtight" , 1 May 2015 (2015-05-01), XP055607435, Retrieved from the Internet: URL:https://web.archive.org/web/2015050104 3528/http://www.alternative-energy-tutoria ls.com/aerobic-composting/anaerobic-compos ting.html [retrieved on 2018-07-31]

## Beschreibung

Die vorliegende Erfindung betrifft ein Verfahren zum Herstellen einer biologisch aktiven, Mikroorganismen enthaltenden wässrigen Lösung bzw. Suspension.

Es ist bekannt, dass Mikroorganismen in großer Vielfalt unsere Umwelt in den unterschiedlichsten Bereichen besiedeln, selbst in unseren Körpern, beispielsweise in unserem Verdauungssystem, vorkommen.

Es ist weiterhin bekannt, dass verschiedentliche Prozesse in Natur und Gesundheit ablaufen, die je nach Art der Besiedelung mit Mikroorganismen, die sich aufgrund der Umweltbedingungen einstellt, einen positiven oder aber einen negativen Einfluss auf den Zustand der Umwelt, aber auch unserer Gesundheit, z.B. unseres Verdauungssystems, haben können. So ist hinsichtlich der Gesundheit beispielsweise bekannt, dass durch die Gabe von Breitbandantibiotika, die neben den mit diesem Medikament zu treffenden Krankheitserregern auch weitere bakterielle Mikroorganismen, insbesondere auch solche der Darmflora treffen und abtöten, die Verdauung zunächst gestört sein kann, bis eine entsprechende ausgewogene und gesunde Darmflora wieder aufgebaut ist.

Auch ist bekannt, dass Gewässer, wie z.B. Auen, Bäche, Tümpel, Teiche, Seen und Flüsse, je nach ihrem Zustand, d.h. beispielsweise der Belastung mit Nährstoffen wie etwa Phosphaten, deren pH-Wert, dem Sauerstoffgehalt und dgl., von ganz unterschiedlichen Mikroorganismen besiedelt sind. Ferner ist bekannt, dass bestimmte Mikroorganismen in einem Milieu in der Lage sind, Schadstoffe abzubauen, um damit die Umweltbedingungen in diesem Milieu wieder zu verbessern. So werden beispielsweise bei der Bekämpfung von Ölpesten regelmäßig solche Bakterien eingesetzt und auf auf dem Gewässer schwimmenden Ölteppichen versprüht, die in der Lage sind, die im Erdöl enthaltenen langkettigen Kohlenwasserstoffe aufzubrechen und zu "verdauen", um somit den Abbau dieses giftigen und umweltschädlichen Stoffes zu beschleunigen.

Mithin ist es also erwünscht, bestimmte Mikroorganismen zu gewinnen bzw. gezielt zu vermehren und in einer biologisch aktiven Weise lagern und zum Einsatz bringen zu können.

Entsprechend besteht Bedarf nach Verfahren zum Herstellen biologisch aktiver, Mikroorganismen enthaltender Ausgangstoffe. Ein solches Verfahren soll mit der Erfindung angegeben werden, wobei hier eine biologisch aktive, Mikroorganismen enthaltende wässrige Lösung bzw. Suspension erlangt werden soll.

Ein solches Verfahren enthält erfindungsgemäß jedenfalls die im Anspruch 1 angegebenen Schritte. Die Ansprüche 2 bis 10 geben vorteilhafte Weiterbildungen des Verfahrens an, wobei in den Ansprüchen 9 und 10 eine zusätzliche Behandlungsstufe beschrieben ist, mittels derer aus einer an sich bereits verwendbaren Suspension eine geänderte, für andere Zwecke verwendbare bzw. anders einsetzbare und wirkende Suspension erhalten werden kann. In Anspruch 11 schließlich ist eine mögliche und bevorzugte Verwendung einer mit einem erfindungsgemäßen Verfahren erhaltenen Lösung bzw. Suspension genannt.

Bei dem Verfahren zum Herstellen einer biologisch aktiven, Mikroorganismen enthaltenden wässrigen Lösung bzw. Suspension gemäß der Erfindung werden in mittels Fermentation umgesetzem Kompost enthaltene Mikroorganismen eingesetzt und durch die verfahrensgemäße Behandlung gezielt vermehrt bzw. in ihren Lebensbedingungen gefördert. In mittels Fermentation umgesetzem Kompost, dies hat der Erfinder festgestellt, sind in besonderem Umfang solche Mikroorganismen enthalten, die einen nutzbringenden Einfluss insbesondere zur Milieuverbesserung von Habitaten und Biotopen (Boden, sowie insbesondere Gewässer) haben. Dies gilt insbesondere für solchen Kompost, der in besonderer Weise hergestellt wird (vgl. hierzu weiter unten stehend).

Diese im mittels Fermentation umgesetzen Kompost enthaltenen Mikroorganismen werden erfindungsgemäß in einer wässrigen Lösung bzw. Suspension wie folgt gewonnen:
Es wird Wasser bereitgestellt. In das bereitgestellte Wasser kann ein feinkörniges, mineralisches oder organisches Material eingegeben werden. Dieses Material wird dann in dem Wasser zum Erhalten einer Suspension vermischt. Bei diesem feinkörnigen Material kann es sich insbesondere um Kaolin, auch als weiße Tonerde, seltener als Kaolinton, bezeichnet. Bei diesem speziellen mineralischen Stoff handelt es sich um ein solches weißfarbiges Mineral, welches besonders feinkörnig mit runden Körnchen in einer Körnungsgröße von weniger als *µ*m natürlich vorkommt. Es kann als feinkörniges Material aber auch Sägespäne oder dergleiches Material zugegeben werden. Das feinkörnige Mineral sollte, wenn es zugegeben wird, in einem auf das jeweilige Volumen bezogenen Anteil von zwischen 1:4 und 1:6, insbesondere von 1:5 dem Wasser zugegeben werden.

Zu der so erhaltenen Suspension wird mittels Fermentation umgesetzer Kompost zugegeben. Die Menge des zugegebenen Komposts (als Volumen gemessen) liegt in Bezug auf das Volumen des eingangs bereitgestellten Wassers bei etwa 1:1000 bis 1:200, kann insbesondere bei etwa 3:1000 liegen. Das Gefäß mit der darin enthaltenen Suspension wird luftdicht verschlossen, um die Suspension so unter Luftabschluss für den Zeitraum von wenigstens einer Woche bei einer Temperatur von weniger als 15°C, bevorzugt sind hier Temperaturen in einem Bereich von 8°C bis 12°C, ruhen zu lassen. Optimale Bedingungen hinsichtlich dieses Ruhenlassens bei den angegebenen Temperaturen finden sich in Kellergewölben, beispielsweise Felsenkellern, es ist aber auch möglich dieses Ruhenlassen in einem Kühlschrank, Kühlraum oder dgl. zu vollziehen. Mit Vorteil sollte das Ruhenlassen jedenfalls weitgehend in Dunkelheit erfolgen.

Am Ende dieses Schrittes des Ruhenlassens erhält man eine Suspension, die in biologisch aktiver und wirksamer Menge Mikroorganismen aus dem Kompost enthält. Dabei hat sich im Zuge des Ruhenlassens das feinkörnige mineralische Material am Grund der Suspension abgesetzt, der wässrige Anteil mit darin enthaltenen Mikroorganismen ist klar und transparent. Der in Versuchsreihen gemessene pH-Wert liegt bei oder unterhalb von 7, das Redoxpotential bei oder oberhalb von 200 mV. Diese Suspension bzw. Lösung kann nun - und wurde von dem Erfinder bereits so verwendet - erfolgreich eingesetzt werden, um in umweltbelasteten Biotopen, insbesondere Gewässern und Böden, eine Verbesserung, insbesondere eine Erhöhung der Gewässer- bzw. Bodenqualität, zu erzielen, indem diese in dem entsprechenden Umfeld ausgebracht wird.

Mit Vorteil wird das eingangs des Verfahrens bereitgestellte Wasser vor dem Hinzugeben des mineralischen Materials abgekocht, um darin enthaltene etwaige unerwünschte Mikroorganismen abzutöten. Das eingangs des Verfahrens bereitgestellte Wasser hat mit Vorteil eine Temperatur von weniger als 15°C, insbesondere eine Temperatur im Bereich von 4°C bis 10°C. Mit solchermaßen temperiertem Wasser haben sich hinsichtlich der Wirksamkeit der mit dem erfindungsgemäßen Verfahren hergestellten Suspension bzw. Lösung die besten Ergebnisse gezeigt.

Ergänzend und zur weiteren Erhöhung der Wirksamkeit kann, vorzugsweise unmittelbar nach dem Hinzugeben des feinkörnigen mineralischen Materials zu dem Wasser, der Suspension Natriumchlorid-Salz, dies insbesondere als Steinsalz oder Meersalz, aber auch als Kristallsalz, zugegeben werden. Dabei sollte die Dosierung nicht zu hoch gewählt werden. Als geeignet hat sich eine - auf das jeweilige Volumen bezogene - Zugabe von Salz zu ursprünglich eingesetztem Wasser in einem Anteil von etwa 1:2000 bis etwa 3:1000, insbesondere von etwa 3:2000 erwiesen.

Eine weitere mögliche Zugabe, die vorzugsweise unmittelbar vor dem Hinzufügen von Kompost zu der Suspension erfolgt, kann in einem Anteil von Aktivkohle, insbesondere Buchenholzkohle, bestehen. Auch hier hat sich erwiesen, dass eine geringe Menge dieses Materials eine Verbesserung der mit dem Verfahren erhaltenen biologisch aktiven, Mikroorganismen enthaltenden wässrigen Lösung ergibt. Hier hat sich eine Zugabe in einer Größenordnung von 1:10000 bis 1:1000, insbesondere von 5:10000 jeweils bezogen auf das Volumen der zugegebenen Aktivkohle im Verhältnis zu dem Volumen des ursprünglich bereitgestellten Wassers als wirkungsvoll erwiesen. Insbesondere sollte die Aktivkohle, vorzugsweise Buchenholzkohle, in Apothekenqualität vorliegen.

Mit den oben bezeichneten Vorgehensweisen wird, wie bereits erwähnt, eine oberhalb der abgesetzten mineralischen Sedimente klare und transparente Lösung erhalten, die bereits Mirkoorganismen in biologisch aktivem Umfang enthält und die so verwendet werden kann.

In einer weiteren Verfahrensstufe kann diese Lösung bzw. Suspension jedoch weiterbehandelt werden, um eine abgeänderte und alternativ oder auch zusätzlich einsetzbare Variante einer biologisch aktiven, Mikroorganismen enthaltenden wässrigen Lösung bzw. Suspension zu erlangen. Dazu wird nach dem Ruhenlassen der Suspension unter Luftabschluss das entsprechende, die Suspension enthaltende Gefäß geöffnet, der Suspension ein kohlenhydrathaltiger Nährstoff, bei dem es sich beispielsweise um Zuckerrohrmelasse handeln kann, zugesetzt, und die Suspension wird unter Wärmezufuhr gären gelassen. Dieser Gärprozess findet dabei insbesondere anaerob statt.

Mit Vorteil wird die Suspension bei einer Temperatur von 25°C bis 40°C, insbesondere bei 30°C gären gelassen. Die Gärdauer beträgt vorteilhafterweise 5 bis 10 Tage, insbesondere 7 Tage.

Am Ende des Gärprozesses erhält man, wie bereits gesagt, eine gegenüber der mit den ersten Verfahrensschritten erhältlichen, bereits biologisch aktiven und verwendbaren Mikroorganismen enthaltenden wässrigen Lösung eine Abwandlung, in der eine andere Zusammensetzung von Mikroorganismen vorherrscht. Messungen des Erfinders haben an Ausführungsbeispielen gezeigt, dass diese Lösung einen geringeren pH-Wert aufweist als die nach der ersten Verfahrensstufe erhaltene Lösung, er liegt bei pH ≤ 4. Auch das ermittelte Redoxpotential ist mit ≤ -200 mV deutlich anders als dasjenige der nach den ersten Verfahrensschritten erhaltenen, bereits verwendbaren Lösung bzw. Suspension.

Für eine spezifische weitere, anwendungsbezogene Rezeptur kann der mit dem erfindungsgemäßen Verfahren enthaltenen Lösung entweder während des Herstellungsprozesses, oder aber nach dem Schritt des Ruhenlassens (ggf. nach dem Schritt des Gärens) weiterer Zusatz beigegeben werden, z.B. Essenzen oder dgl.

Mit Eingabe von erfindungsgemäß gewonnenen Lösungen nach dem Ruhenlassen (klar und transparent) bzw. nach dem Gärprozess (trüb wegen der noch enthaltenen kohlenhydrathaltiger Nährstoffe) ist es dem Erfinder bereits gelungen, in bedenklichem ökologischen Zustand befindliche (umgekippte) Gewässer hinsichtlich ihrer Wasserqualität deutlich zu verbessern. Die so eingebrachten Mikroorganismen waren in der Lage, in dem Milieu des Gewässers enthaltene schädliche Substanzen abzubauen und damit die Wasserqualität deutlich zu erhöhen. Auch konnten verbesserte Erträge von Kulturpflanzen erzielt werden, wenn der Boden, auf dem diese gezogen wurden, mit der erfindungsgemäßen Suspension (in einer der beiden Varianten) behandelt worden war. Auch, hier nicht geschützte Anwendungen beim Menschen, z.B. bei äußerlicher Anwendung oder auch innerlicher Einnahme sind denkbar, so z.B. zur (begleitenden) Behandlung von Hauterkrankungen wie Neurodermitis.

Nachfolgend wird das erfindungsgemäße Verfahren anhand einer beispielhaften Rezeptur noch weiter erläutert.

Eine erste Variante einer erfindungsgemäßen biologisch aktiven, Mikroorganismen enthaltenen wässrigen Lösung bzw. Suspension wurde erhalten wie folgt:
Es wurden 10 I Wasser mit Trinkwasserqualität abgekocht, dabei für etwa 10 Minuten leicht sieden gelassen, und anschließend abgekühlt. Nach Abkühlen auf Raumtemperatur wurde das Wasser in geeigneter Weise, z.B. einem Kühlschrank, auf eine Temperatur zwischen 4°C und 10°C abgekühlt und in eine dieses Volumen fassende Glasflasche gefüllt. Dem Wasser wurde anschließend feinkörniges Kaolin in einem Volumen von 2 I beigegeben. Die so gewonnene Mischung wurde zum Herstellen einer Suspension gut durchmischt. Anschließend wurde Natriumchloridsalz in einem Volumen von 14 ml als Kristallsalz eingemischt, wobei auch Steinsalz oder Meersalz Verwendung finden können. In einem darauffolgenden Schritt wurde feingemahlene Buchenholzkohle in Apothekenqualität in einem Volumen von 5 ml in die Suspension eingemischt. Zuletzt wurde Kompost in einem Volumen von 28 ml eingemischt, das Glasgefäß wurde luftdicht verschlossen und unter Lagerung des Gefäßes in kühler Atmosphäre bei 8°C bis 12°C wurde die Mischung ruhen gelassen, wobei eine Reifung in Form einer kalten Fermentation stattfand.

Als Kompost wurde ein eigens für diesen Herstellungsprozess hergestellter Kompost verwendet, dessen Herstellung weiter unten stehend noch einmal gesondert beschrieben wird.

Am Ende des oben beschriebenen Verfahrens, nach dem Ruhenlassen, wurde oberhalb der abgesetzten Kaolinsedimente eine klare wässrige Lösung erhalten, die sich als biologisch aktiv erwiesen hat und Mikroorganismen enthielt. Der pH-Wert dieser Lösung lag bei oder unterhalb von 7, ein gemessenes Redoxpotential von mehr als 200 Millivolt konnte festgestellt werden. Diese Lösung konnte mühelos über längere Zeiträume gelagert werden und eignete sich zum Einbringen der darin enthaltenen Mikroorganismen in belastete Lebensräume, insbesondere Gewässer, um damit den Abbau schädlicher Bestandteile zu fördern und die Qualität der Lebensräume zu verbessern.

Aus dieser Lösung lässt sich gemäß einer Erweiterung des Verfahrens nach der nachfolgend geschilderten Rezeptur eine weitere Variante einer erfindungsgemäßen Lösung bzw. Suspension herstellen. Hierzu wurden 12 I der in dem ersten Verfahren hergestellten Suspension (also inklusive des enthaltenen Kaolins) als Ausgangsmaterial herangezogen. Dieser Menge wurde Zuckerrohrmelasse, insbesondere solcher aus ökologischem Anbau, in einer Menge von 200 ml zugegeben. Diese Suspension wurde durch Schütteln oder Rühren gut durchmengt. Das diese Suspension enthaltende Gefäß wurde mit einem Gärspund verschlossen und in das Wasserbad eines Fermenters gestellt. Das Wasser des Wasserbades wurde auf einer Temperatur von 30°C gehalten, diese Mischung wurde über sieben Tage gären gelassen.

Am Ende des Gärprozesses ergab sich eine trübe und bräunlich gefärbte wässrige Lösung bzw. Suspension mit einem deutlich geringeren pH-Wert von pH < 4 und einem gemessenen Redoxpotential von etwa - 200 mV. Auch diese Lösung zeigte sich als sehr wirksam in der Verwendung für die Verbesserung von Lebensräumen, insbesondere Gewässern, indem die darin enthaltenen spezifischen Mikroorganismen den Abbau von Schadstoffen beschleunigten und somit ein ökologisch günstiges Milieu unterstützte.

Eine bevorzugte Möglichkeit den in dem oben geschilderten Verfahren verwendeten Kompost herzustellen wird nun geschildert.

Die Herstellung des Komposts beruht auf einer Fermentation anstelle eines sonst häufig anzutreffenden Fäulnisprozesses. Der Umstand, dass hier die Ausgangsstoffe im Wege der Fermentation umgesetzt werden, ist von wesentlicher Bedeutung, da nur hierbei die gewünschte Fauna an Mikroorganismen erhalten wird; bei Fäulnis bildet sich eine andere und eben nicht günstige Mikrofauna.

Der Fermentkompost kann in sieben Schritten hergestellt werden wie folgt:
- Pflanzliche und tierische Abfälle (z.B. Küchen- und Schlachtabfälle) werden bei 10-20°C unter Luftabschluss nach Zugabe von Mikroorganismen kalt fermentiert (insbesondere nach dem sog. Bokashi-Verfahren).
- Weitere pflanzliche und tierische Rohstoffe (organisches Material, auch Fäkalmaterial) wird gesammelt.
- Es wird dem weiteren pflanzlichen und tierischen Rohstoffen Wasser zugegeben zur Einstellung eines gut durchfeuchteten Gemenges, ohne dieses zu verflüssigen.
- Das im ersten Schritt erhaltene Material und das durchfeuchtete Gemenge aus den weiteren pflanzlichen und tierischen Rohstoffen wird zusammengeführt und mit einem Zusatz von reifem Fermentkompost (insbesondere solchem, wie er nach diesem Verfahren hergestellt wird) geimpft.
- Das so gewonnene Ausgangsmaterial wird in einer Miete aufgeschichtet, die insbesondere glockenförmig, kugelabschnittförmig oder abgerundet kegelförmig gebildet ist.
- Die einsetzende Fermentation wird nun durch Regelung der Luft- und Wasserzufuhr derart gesteuert, dass sie im Bereich um 65°C abläuft, d.h. dass die Kerntemperatur in der Miete ca. 65°C beträgt.
- Der Kompost wird nun während einer Reifedauer von ca. 6 Monaten reifen gelassen und dabei mehrfach umgesetzt:
   - Nach dem Aufsetzten der Miete steigt die Temperatur in dem Material nach einigen Tagen auf etwa 65°C. Nach einiger Zeit fällt die Temperatur ab.
   - Wenn der Kompost nach ca. 3 Wochen die Temperatur von 55°C unterschreitet, wird die Miete das erste Mal umgesetzt. Die Temperatur steigt dann wieder an und erreicht ein zweites Mal die Temperatur von ca. 65°C, wobei auch hier die Temperatur nach einiger Zeit erneut abfällt.
   - Wenn der Kompost dann die Temperatur von 40°C unterschreitet, wird er ein zweites Mal umgesetzt.
   - Ein drittes Umsetzen erfolgt, wenn in der Miete die Temperatur von 30°C unterschritten wird.
   - Ein viertes Umsetzen erfolgt, wenn in der Miete die Temperatur von 25°C unterschritten wird.
   - Der Fermentkompost ist dann reif, wenn sich seine Temperatur der normalen Bodentemperatur annähert und sich eine gleichbleibende Temperatur von zwischen 8°C und 16°C eingestellt hat. Dies ist in der Regel nach dem vierten Umsetzen der Miete der Fall.

Das organische Material in der Miete sollte dabei gut durchmengt werden und nach Möglichkeit in seinen Bestandteilen vielfältig und von unterschiedlichsten Arten der Lebewesen, denen sie entstammen, herrühren. Es gilt dabei die grundsätzliche Faustformel: Je homogener die Mischung und je artenreicher das Spektrum des organischen Materials, desto besser das Ergebnis. Dabei sollen aber etwa krankheitsbefallene organische Reste, wie z.B. Grünabfälle erkrankter Pflanzen, oder auch schadstoffbelastete Anteile, in der Mitte der Miete eingebracht werden. Wie bereits erwähnt ist wichtig, dass der Kompost fermentiert wird. Dies geschieht bei Temperaturen von mindestens 55°C, insbesondere bei 65°C, bei denen die sog. Heißfermentation (auch mesotherme Phase genannt) abläuft. Der Umstand, dass das organische Material über Fermentationsprozesse kompostiert wird und keine Fäulnisprozesse beteiligt sind, ist bei diesem Vorgehen deshalb von besonderer Wichtigkeit. Denn hier bestimmen Pilze den Umsetzungsprozess. Es existieren bei diesen Bedingungen solche Aerobier, die antibiotisch wirkende Stoffe produzieren, die in der Lage sind Schädlinge wie Wurmeier, krankheitserregende Keime und selbst Sporenbildner wie Milzbrandsporen sicher zu vernichten. Deshalb soll in der Miete vor dem ersten Umsetzen unbedingt eine Temperatur von 55°C oder darüber, insbesondere 65°C erreicht werden. Dies geschieht typischerweise nach dem ersten Aufsetzen der Miete nach einigen Tagen und muss mindestens drei Wochen andauern, muss dabei mit einer Temperaturmessung überprüft werden.

Eine noch verbesserte Wirkung ergibt sich, wenn zur Durchlüftung ein Rohr in etwa mittig und vertikal verlaufend in die Kompostmiete gesteckt wird. Durch den Kamineffekt wird erreicht, dass hier eine intensivere Durchlüftung des organischen Materials und eine entsprechende Versorgung der aeroben Mikroorganismen erhalten werden. Besonders gute Ergebnisse konnte der Erfinder hier erzielen mit einem Rohr, welches hinsichtlich der Länge auf die Frequenzlage der sog. Erdfrequenz, auch Erdenton genannt, (nach mehrfachem Oktavieren auf 68,051 Hz) angepasst und dazu in verschiedenen Längen durch Querschnittsverjüngungen unterteilt war. Die Länge dieses Rohres betrug 2329 mm. Der Außendurchmesser (der indes für die Wirkung nicht exakt so bemessen sein muss) betrug 140 mm mit einer Wandstärke von 12,7 mm. Das Rohr war aus Polyethylen (PE) gebildet, insbesondere aus PE-HD. Die Unterteilung der verschiedenen durch die Querschnittsverjüngungen gebildeten Kammern war dann wie folgt:
Eine in den Kompost eingebrachte unterste Kammer mit seitlichen Öffnungen in der Rohrwand war 348 mm lang. Sämtliche sich daran anschließenden Kammern waren ohne Durchbrechungen der Rohrwand und in dieser Reihenfolge 229, 313, 229, 439, 229, 313 und 229 mm lang. Dadurch war das Rohr auf die harmonischen Obertöne des Erdentons abgestimmt.

Die oben als Ausführungsbeispiele dargestellten Rezepturen sind rein beschreibende und beschränken die Erfindung in ihrem Umfang nicht.

## Patentansprüche

1. Verfahren zum Herstellen einer biologisch aktiven, Mikroorganismen enthaltenden wässrigen Lösung bzw. Suspension mit folgenden Schritten:
i) Bereitstellen von Wasser;
ii) Hinzugeben von mittels Fermentation umgesetzten Kompost zum Bilden einer Suspension, wobei das Verhältnis des Volumens des hinzugegebenen Komposts zum Volumen des bereitgestellten Wassers 1:1000 bis 1:200 beträgt;
iii) Ruhenlassen der Suspension unter Luftabschluss für wenigstens eine Woche bei einer Temperatur von weniger als 15°C.

2. Verfahren nach Anspruch 1, **dadurch gekennzeichnet, dass** das Wasser vor dem Bereitstellen abgekocht wird.

3. Verfahren nach einem der vorhergehenden Ansprüche, **dadurch gekennzeichnet, dass** dem Wasser vor dem Schritt iii), insbesondere zwischen den Schritten i) und ii), ein feinkörniges mineralisches oder organisches Material zugegeben und dieses Material in dem Wasser zum Erhalten einer Suspension vermischt wird.

4. Verfahren nach Anspruch 3, **dadurch gekennzeichnet, dass** als feinkörniges mineralisches Material Kaolin verwendet wird.

5. Verfahren nach einem der vorhergehenden Ansprüche, **dadurch gekennzeichnet, dass** das in Schritte i) bereit gestellte Wasser eine Temperatur von weniger als 15°C aufweist, vorzugsweise eine Temperatur im Bereich von 4°C bis 10°C.

6. Verfahren nach einem der vorhergehenden Ansprüche, **dadurch gekennzeichnet, dass** die Suspension in Schritt iii) bei einer Temperatur von 8°C bis 12°C ruhen gelassen wird, vorzugsweise über einen Zeitraum von wenigstens einer Woche.

7. Verfahren nach einem der vorhergehenden Ansprüche, **dadurch gekennzeichnet, dass** dem Wasser und/oder der Suspension vor dem Ruhenlassen Natriumchlorid-Salz, insbesondere als Steinsalz oder Meersalz, zugegeben wird, vorzugsweise unmittelbar nach Schritt i) oder unmittelbar nach der Zugabe eines feinkörnigen organischen oder mineralischen Materials.

8. Verfahren nach einem der vorhergehenden Ansprüche, **dadurch gekennzeichnet, dass** dem Wasser und/oder der Suspension vor dem Ruhenlassen Aktivkohle, insbesondere Buchenholzkohle, zugegeben wird, vorzugsweise unmittelbar vor Schritt ii).

9. Verfahren nach einem der vorhergehenden Ansprüche, **dadurch gekennzeichnet, dass** nach Schritt iii) der Suspension ein kohlehydrathaltiger Nährstoff, insbesondere Zuckerrohrmelasse, zugesetzt und die Suspension unter Wärmezufuhr gären gelassen wird.

10. Verfahren nach Anspruch 9, **dadurch gekennzeichnet, dass** die Suspension bei einer Temperatur von 25°C bis 40°C, insbesondere bei 30°C, für 5 bis 10, insbesondere 7, Tage gären gelassen wird.

11. Verwendung einer nach dem Verfahren gemäß einem der vorhergehenden Ansprüche gewonnenen Lösung bzw. Suspension für die Verbesserung der Qualität von Gewässern, Böden, Werkstoffen durch Einbringen der Suspension in das jeweilige System.

## Claims

1. A method for preparing a biologically active aqueous solution or suspension containing microorganisms, comprising the steps of:
i) Providing water;
ii) Adding compost converted by fermentation to form a suspension, where the ratio of the volume of compost added to the volume of water provided is 1:1000 to 1:200
iii) Allow the suspension to rest in the absence of air for at least one week at a temperature of less than 15°C.

2. A method according to claim 1, **characterised in that** the water is boiled before being made available,

3. A method according to any of the preceding claims, **characterised in that** a fine-grained mineral or organic material is added to the water before step iii), in particular between steps i) and ii), and this material is mixed in the water to obtain a suspension.

4. A method according to claim 3, **characterised in that** kaolin is used as fine-grained mineral material.

5. A method according to any of the preceding claims, **characterised in that** the water provided in step i) has a temperature of less than 15°C, preferably a temperature in the range of 4°C to 10°C.

6. A method according to any of the preceding claims, **characterised in that** the suspension in step iii) is allowed to rest at a temperature of 8°C to 12°C, preferably for a period of at least one week.

7. A method according to any of the preceding claims, **characterised in that** sodium chloride salt, in particular as rock salt or sea salt, is added to the water and/or the suspension before it is left to rest, preferably immediately after step i) orimmediately after the addition of a fine-grained organic or mineral material.

8. A method according to any of the preceding claims, **characterised in that** activated carbon, in particular beech charcoal, is added to the water and/or the suspension before it is allowed to rest, preferably immediately before step ii).

9. A method according to one of the preceding claims, **characterised in that** after step iii) a carbohydrate-containing nutrient, in particular sugar cane molasses, is added to the suspension and the suspension is allowed to ferment under heat supply.

10. A method according to claim 9, **characterised in that** the suspension is allowed to ferment at a temperature of 25°C to 40°C, in particular at 30°C, for 5 to 10, in particular 7, days.

11. The use of a solution or suspension obtained by the method according to one of the previous claims for the improvement of the quality of water, soil, materials by introducing the suspension into the respective system.

## Revendications

1. Procédé de préparation d'une solution ou suspension aqueuse biologiquement active contenant des microorganismes, comprenant les étapes suivantes
i) Fourniture d'eau;
ii) Ajout de compost converti par fermentation pour former une suspension, le rapport entre le volume de compost ajouté et le volume d'eau fourni étant compris entre 1:1000 et 1:200
iii) Laisser la suspension reposer en l'absence d'air pendant au moins une semaine à une température inférieure à 15°C.

2. Procédé selon la revendication 1, **caractérisé par le fait que** l'eau est portée à ébullition avant mise à disposition,

3. Procédé selon l'une des revendications précédentes, **caractérisé en ce qu'**une matière minérale ou organique à grain fin est ajoutée à l'eau avant l'étape iii), en particulier entre les étapes i) et ii), et cette matière est mélangée dans l'eau pour obtenir une suspension.

4. Procédé selon la revendication 3, **caractérisé en ce que** le kaolin est utilisé comme matière minérale à grain fin.

5. Procédé selon l'une des revendications précédentes, **caractérisé en ce que** l'eau fournie à l'étape i) a une température inférieure à 15°C, de préférence une température comprise entre 4°C et 10°C.

6. Procédé selon l'une des revendications précédentes, **caractérisé en ce que** la suspension de l'étape iii) est laissée au repos à une température de 8°C à 12°C, de préférence pendant une période d'au moins une semaine.

7. Procédé selon l'une des revendications précédentes, **caractérisé en ce que** le sel de chlorure de sodium, en particulier sous forme de sel gemme ou de sel marin, est ajouté à l'eau et/ou à la suspension avant qu'elle ne soit mise au repos, de préférence immédiatement après l'étape i) ou immédiatement après l'ajout d'une matière organique ou minérale à grain fin.

8. Procédé selon l'une des revendications précédentes, **caractérisé en ce que** du charbon actif, en particulier du charbon de hêtre, est ajouté à l'eau et/ou à la suspension avant qu'on le laisse reposer, de préférence immédiatement avant l'étape ii).

9. Procédé selon l'une des revendications précédentes, **caractérisé en ce qu'**après l'étape iii), un nutriment contenant des glucides, en particulier de la mélasse de canne à sucre, est ajouté à la suspension et la suspension est laissée fermenter sous apport de chaleur.

10. Procédé selon la revendication 9, **caractérisé en ce que** l'on laisse la suspension fermenter à une température de 25°C à 40°C, en particulier à 30°C, pendant 5 à 10, en particulier 7, jours.

11. Utilisation d'une solution ou d'une suspension obtenue par le procédé selon l'une des revendications précédentes pour l'amélioration de la qualité de l'eau, du sol, des matériaux en introduisant la suspension dans le système respectif.
